(19) Europäisches Patentamt
European Patent Office
Office européen des brevets

(11) Veröffentlichungsnummer: **0 303 933**
**A2**

# (12) EUROPÄISCHE PATENTANMELDUNG

(21) Anmeldenummer: **88112884.7**

(22) Anmeldetag: **08.08.88**

(51) Int. Cl.4: **C07D 493/22 , A01N 43/90 , A61K 31/35 , //(C07D493/22, 313:00,311:00,311:00,307:00)**

Die Anmeldung wird, wie ursprünglich eingereicht, unvollständig veröffentlicht (Art. 93 (2) EPÜ). Die Stelle der Beschreibung oder der Patentansprüche, die offensichtlich eine Auslassung enthält, ist als Lücke an der entsprechenden Stelle ersichtlich.

(30) Priorität: **19.08.87 DE 3727648**

(43) Veröffentlichungstag der Anmeldung: **22.02.89 Patentblatt 89/08**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Röben, Wolfgang, Dr.**
**Strässschen Siefen 30**
**D-5060 Bergisch-Gladbach 2(DE)**
Erfinder: **Stendel, Wilhelm, Dr.**
**In den Birken 55**
**D-5600 Wuppertal 1(DE)**
Erfinder: **Andrews, Peter, Dr.**
**Gellertweg 2**
**D-5600 Wuppertal 1(DE)**

## (54) Neue Avermectin Derivate, Verfahren zu ihrer Herstellung und ihre Verwendung.

(57) Die vorliegende Erfindung betrifft neue Avermectin Derivate der Formel I

EP 0 303 933 A2

Xerox Copy Centre

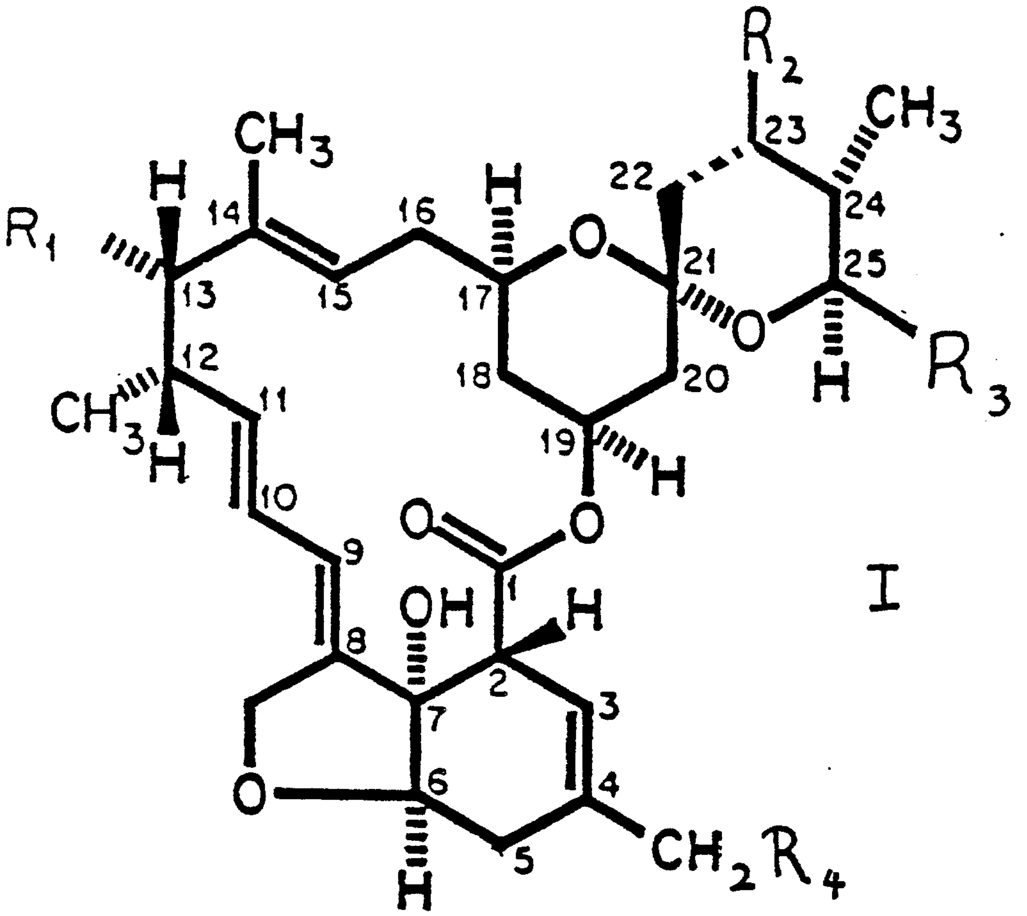

I

in welcher

$R^1$ für Wasserstoff, OH, $C_{1-5}$-Alkanoyloxy, α-L-Oleandrosyloxy, α-L-Oleandrosyl-α-L-oleandrosyloxy, 4′-$C_{1-5}$-Alkanoyl-α-L-Oleandrosyloxy, 4″-$C_{1-5}$-Alkanoyl-α-L-Oleandrosyl-α-L-oleandrosyloxy steht,

$R^2$ für Wasserstoff, OH, $C_{1-5}$-Alkanoyloxy steht, $R^2$ steht für Wasserstoff, wenn zwischen C22 und C23 eine Doppelbindung ist,

$R^3$ für geradkettiges oder verzweigtes Alkyl oder Alkenyl steht,

$R^4$ für Wasserstoff, OH, $C_{1-5}$-Alkanoyloxy, Heterocyclylcarbonyloxy steht, die Bindung zwischen den C-Atomen C22 und C23 eine Einfach- oder eine Doppelbindung ist und die Doppelbindung des Cyclohexenringes zwischen den C-Atomen C3 und C4 oder zwischen den C-Atomen C4 und C5 stehen kann,

Verfahren zu ihrer Herstellung, Zwischenprodukte zur Durchführung dieser Verfahren und ihre Verwendung als Parasitizide.

## Neue Avermectin Derivate, Verfahren zu ihrer Herstellung und ihre Verwendung

Die vorliegende Erfindung betrifft neue Avermectin Derivate, Verfahren zu ihrer Herstellung, Zwischenprodukte zur Durchführung dieser Verfahren und ihre Verwendung als Parasitizide.

Avermectine sind breit wirksame Ekto- und Endoparasitizide. Ein Derivat mit dem Freinamen Ivermectin ist als Parasitizid bei Tieren im Handel. Seine Wirkung gegen Zecken ist jedoch nicht voll befriedigend.

1. Es wurden die neuen Verbindungen der Formel I gefunden

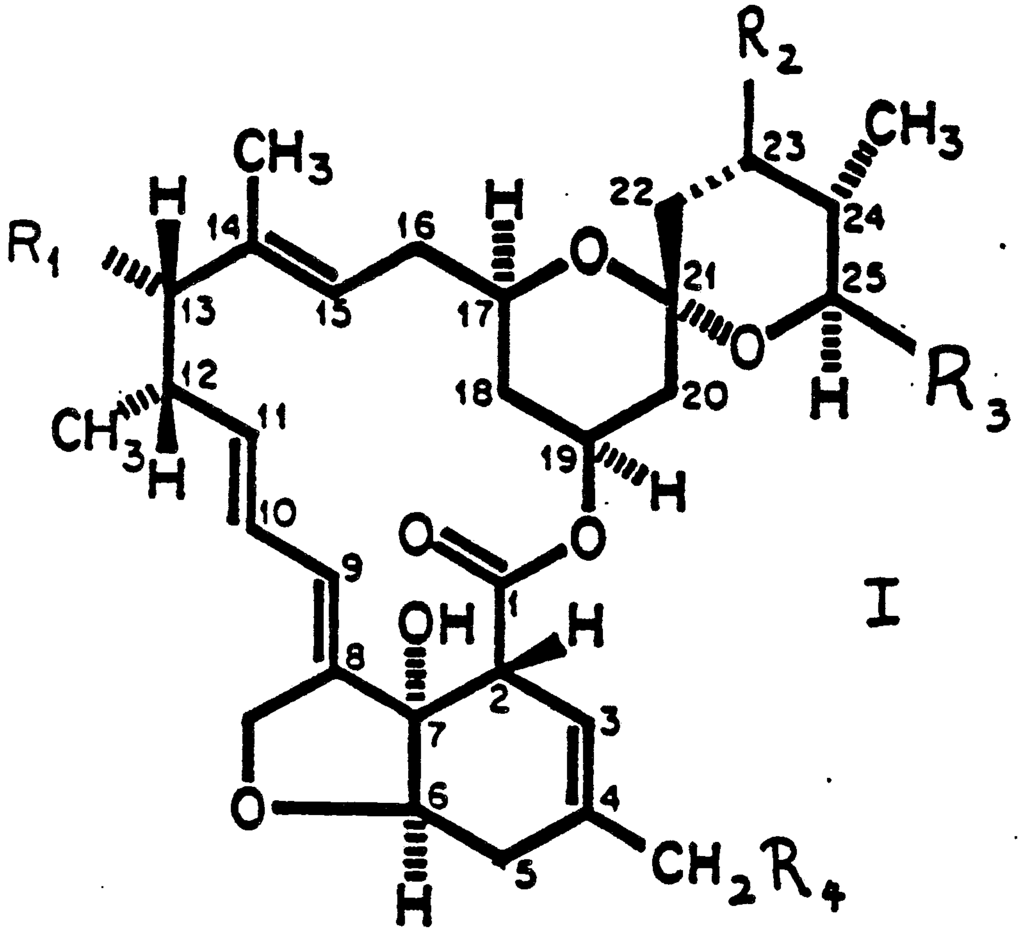

I

in welcher

$R^1$ für Wasserstoff, OH, $C_{1-5}$-Alkanoyloxy, α-L-Oleandrosyloxy, α-L-Oleandrosyl-α-L-oleandrosyloxy, 4′-$C_{1-5}$-Alkanoyl-α-L-Oleandrosyloxy, 4″-$C_{1-5}$-Alkanoyl-α-L-Oleandrosyl-α-L-oleandrosyloxy steht,

$R^2$ für Wasserstoff, OH, $C_{1-5}$-Alkanoyloxy steht, $R^2$ steht für Wasserstoff, wenn zwischen C22 und C23 eine Doppelbindung ist,

$R^3$ für geradkettiges oder verzweigtes Alkyl oder Alkenyl steht,

$R^4$ für Wasserstoff, OH, $C_{1-5}$-Alkanoyloxy, Heterocyclylcarbonyloxy steht, die Bindung zwischen den C-Atomen C22 und C23 eine Einfach- oder eine Doppelbindung ist und die Doppelbindung des Cyclohexenringes zwischen den C-Atomen C3 und C4 oder zwischen den C-Atomen C4 und C5 stehen kann.

2. Es wurde ein Verfahren zur Herstellung der Verbindungen der Formel I gefunden

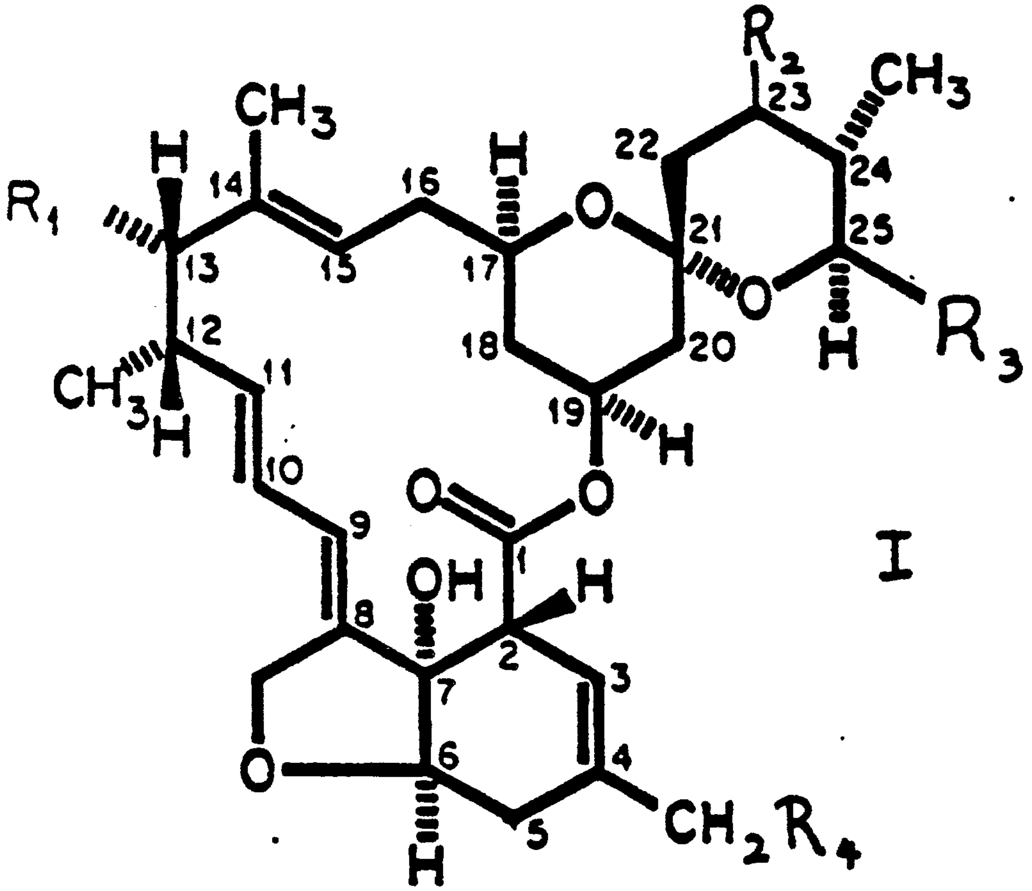

I

in welcher
$R^1$ für Wasserstoff, OH, $C_{1-5}$-Alkanoyloxy, α-L-Oleandrosyloxy, α-L-Oleandrosyl-α-L-oleandrosyloxy, 4'-$C_{1-5}$-Alkanoyl-α-L-Oleandrosyloxy, 4''-$C_{1-5}$-Alkanoyl-α-L-Oleandrosyl-α-L-oleandrosyloxy steht,
$R^2$ für Wasserstoff, OH, $C_{1-5}$-Alkanoyloxy steht, $R^2$ steht für Wasserstoff, wenn zwischen C22 und C23 eine Doppelbindung ist,
das dadurch gekennzeichnet ist, daß man Verbindungen der Formel II

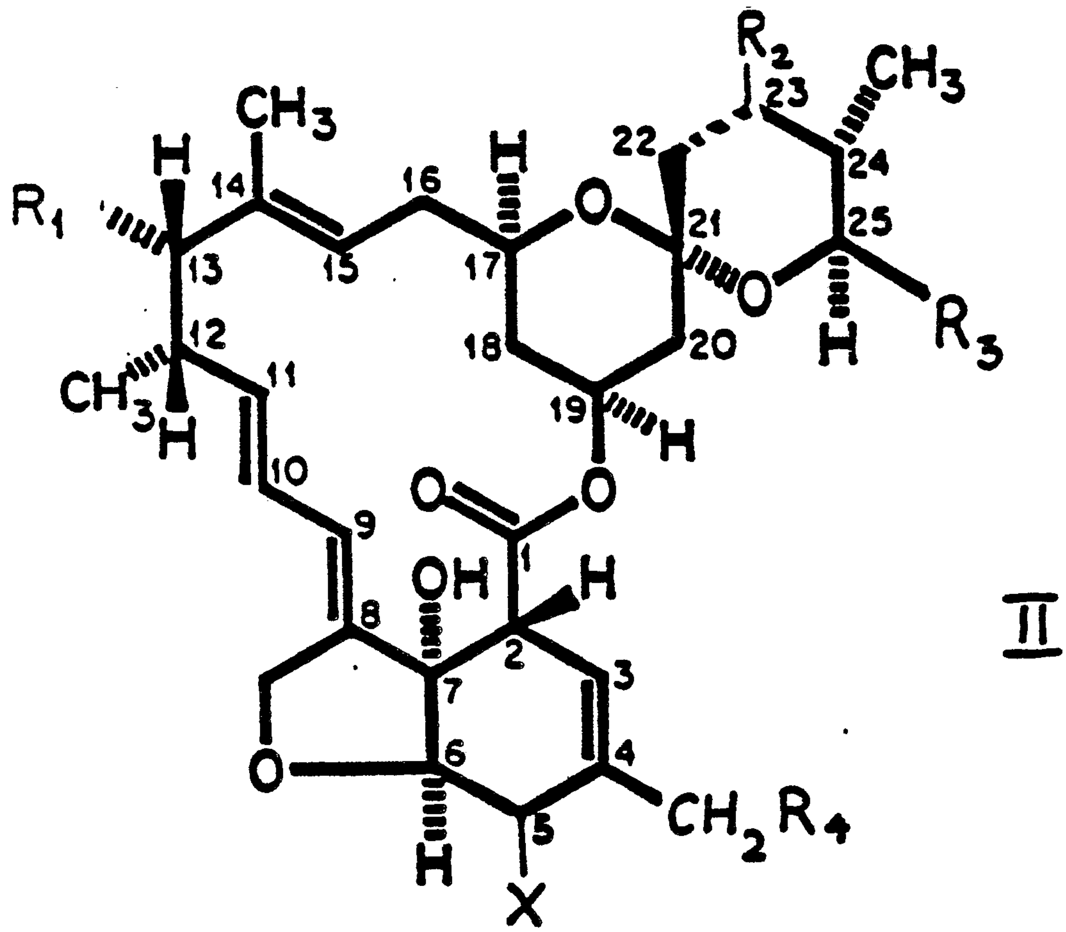

II

in welcher
$R^1$, $R^2$, $R^3$, $R^4$ die oben angegebene Bedeutung haben und
X für Halogen steht
reduziert.

3. Neue Verbindungen der Formel II

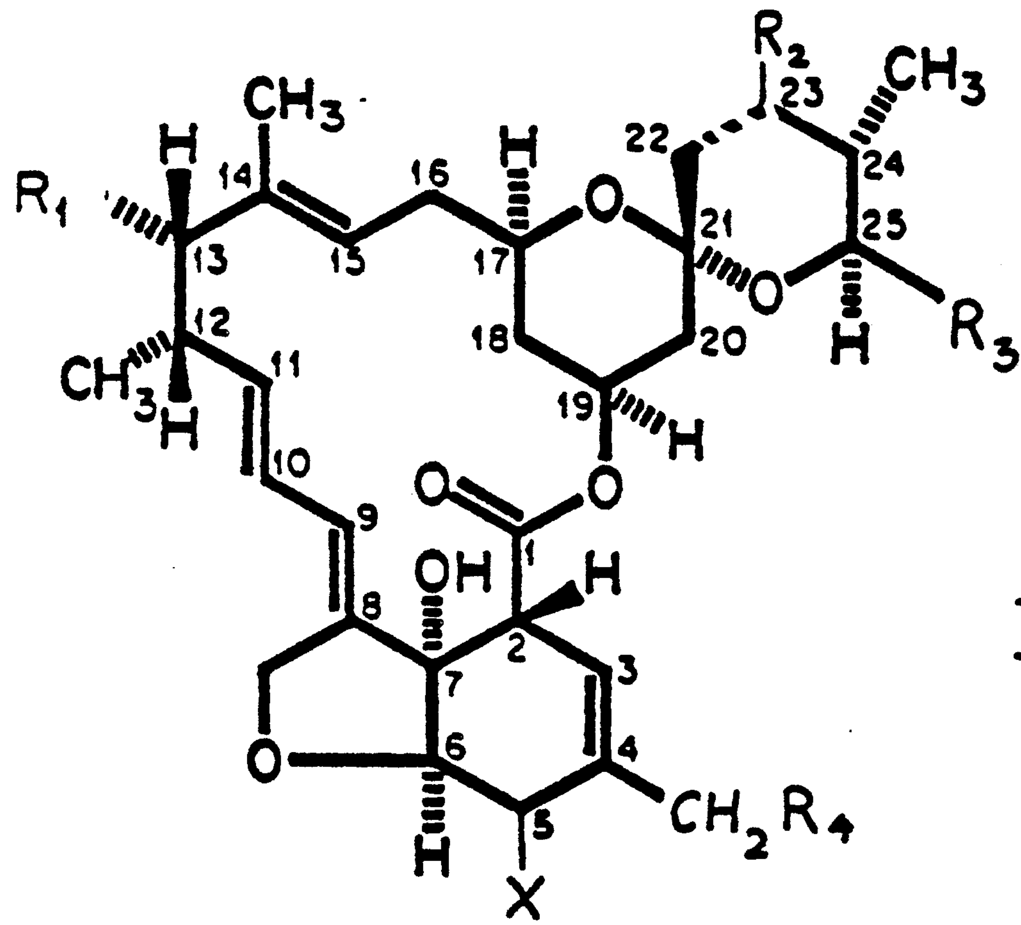

II

in welcher

$R^1$ für Wasserstoff, OH, $C_{1-5}$-Alkanoyloxy, α-L-Oleandrosyloxy, α-L-Oleandrosyl-α-L-oleandrosyloxy, 4′-$C_{1-5}$-Alkanoyl-α-L-Oleandrosyloxy, 4″-$C_{1-5}$-Alkanoyl-α-L-Oleandrosyl-α-L-oleandrosyloxy steht,

$R^2$ für Wasserstoff, OH, $C_{1-5}$-Alkanoyloxy steht, $R^2$ steht für Wasserstoff, wenn zwischen C22 und C23 eine Doppelbindung ist,

$R^3$ für geradkettiges oder verzweigtes Alkyl oder Alkenyl steht,

$R^4$ für Wasserstoff, OH, $C_{1-5}$-Alkanoyloxy, Heterocyclylcarbonyloxy steht, die Bindung zwischen den C-Atomen C22 und C23 eine Einfach- oder eine Doppelbindung ist und die Doppelbindung des Cyclohexenringes zwischen den C-Atomen C3 und C4 oder zwischen den C-Atomen C4 und C5 stehen kann,

X für Halogen steht.

4. Verfahren zur Herstellung der Verbindung der Formel II gemäß 2 (oben) dadurch gekennzeichnet, daß man Verbindungen der Formel II in welcher

X für den Rest $-OSO_2-R^5$ steht wobei

$R^5$ für Alkyl, Halogenalkyl, Aryl, substituiertes Aryl steht,

mit Halogeniden umsetzt, oder Verbindungen der Formel II, in welcher X für OH steht, mit Arylsulfonylhalogeniden in Gegenwart von Dimethylaminopyridin umsetzt.

Von den Avermectinen war bekannt, daß durch Änderung der Substitution am C-Atom 5 die akarizide Wirkung der Verbindungen verringert wird (R.A. Dybar et. al. 1984 British Crop Protection Conference Pests and Diseases 9B-3 p. 947 - 54). Überraschenderweise wurde gefunden, daß die am C-Atom 5 unsubstituierten Derivate der Avermectine hervorragende Wirkung gegen Zecken aufweisen. Sie lassen sich daher bevorzugt als Ekto- und Endoparasitizide bei Tieren einsetzen.

Bevorzugt sind Verbindungen der Formel I in welcher

$R^1$ für Wasserstoff, OH, Acetoxy, α-L-Oleandrosyl-α-L-oleandrosyloxy steht,

$R^2$ für Wasserstoff, OH, Acetoxy steht,

$R^3$ für $C_{1-4}$-Alkyl, insbesondere Methyl, Ethyl, Isopropyl, sec. Butyl, $C_{2-8}$-Alkenyl, insbesondere 2-Butenyl-(2), 4-Methyl-pent-2-enyl(2), 4-Methyl-hex-2-enyl(2),

$R^4$ für Wasserstoff steht,

zwischen C22 und C23 eine Einfachbindung steht,
zwischen C3 und C4 eine Doppelbindung steht,
zwischen C4 und C5 eine Einfachbindung steht.

Besonders bevorzugt sind Verbindungen der Formel I in welcher
$R^1$ für α-L-Oleandrosyl-α-L-oleandrosyloxy steht,
$R^2$ für Wasserstoff steht,
$R^3$ für Isopropyl oder sec.-Butyl steht,
$R^4$ für Wasserstoff steht,
zwischen C22 und C23 eine Einfachbindung steht,
zwischen C3 und C4 eine Doppelbindung steht,
zwischen C4 und C5 eine Einfachbindung steht.

Die Verbindungen der Formel I werden erhalten indem man Verbindungen der Formel II reduziert. Als Reduktionsmittel dienen bevorzugt organische Zinnhydride wie z.B. Tributylzinnhydrid, Tricyclohexylzinnhydrid, Triphenylzinnhydrid in Gegenwart von Radikalbildenden Katalysatoren wie z.B. Peroxiden oder Azobisisobutyronitril.

Die Reaktion wird unter Feuchtigkeitsausschluß in organischen Lösungsmitteln durchgeführt, die unter den Reaktionsbedingungen inert sind. Dazu gehören aliphatische und aromatische Kohlenwasserstoffe, Hexan, Cyclohexan, Benzol, Toluol, Xylol, cyclische Ether wie Tetrahydrofuran, Dioxan, ferner Dimethylformamid.

Es wird bei Temperaturen zwischen 20 und 100 °C, bevorzugt zwischen 40 und 60 °C, gearbeitet.

Es wird bevorzugt bei Normaldruck gearbeitet. Nach erfolgter Umsetzung wird der Ansatz chromatographisch gereinigt.

Verbindungen der Formel II sind neu. Bevorzugt sind Verbindungen der Formel II in welcher die Substituenten $R^1$, $R^2$, $R^3$, $R^4$ die bei den Verbindungen der Formel I angegebene bevorzugten Bedeutungen besitzen und X für Chlor oder Brom steht.

Verbindungen der Formel II in denen X für Halogen steht werden erhalten indem man Verbindungen der Formel II in denen X für den Rest der Formel $-O-SO_2-R^5$ steht mit Halogeniden umsetzt. Solche Verbindungen sind teilweise bekannt oder können analog zu bekannten Verfahren hergestellt werden (CA 101; 23 232 v, CA 99; 194 690 n, CA 98; 89 075 x).

Bevorzugt werden als Ausgangsverbindungen Verbindungen der Formel II eingesetzt in denen X für $OSO_2R^5$ steht und $R^5$ für p-Toluol oder $C_{1-6}$-Alkyl steht.

Bevorzugt werden als Halogenide Alkalihalogenide wie KBr, KCl, quartäre Ammoniumhalogenide, Halogenwasserstoffkomplexe von gegebenenfalls substituierten Pyridinen eingesetzt.

Die Reaktion wird in Verdünnungsmitteln durchgeführt, die unter den Reaktionsbedingungen inert sind. Dazu gehören gegebenenfalls halogenierte Kohlenwasserstoffe wie Petrolether, Benzin, Cyclohexan, Toluol, Xylol, Chloroform, Dichlormethan, o-Dichlorbenzol, Ether wie Diethylether, Tetrahydrofuran, Dioxan, Ketone wie Aceton, Ester wie Essigsäureethylester, DMSO, DMF.

Die Reaktion wird zwischen -40 °C und 80 °C, vorzugsweise zwischen 20 °C und 50 °C durchgeführt.

Es wird auf 1 Mol Verbindung der Formel II ca. 1-5 Mol, bevorzugt 1-2 Mol, Halogenid eingesetzt.

Nach Beendigung der Reaktion wird das Reaktionsgemisch bevorzugt chromatographisch getrennt.

Verbindungen der Formel II, in denen X für Cl steht, lassen sich auch herstellen, indem man die bekannten Verbindungen der Formel II in denen X für OH steht, mit Arylsulfonylhalogeniden, bevorzugt p-Toluolsulfonylchlorid oder o-Nitrobenzolsulfonylchlorid in Gegenwart von Dimethylaminopyridin und in Gegenwart eines Verdünnungsmittels umsetzt. Geeignete Verdünnungsmittel sind die weiter oben beschriebenen Verdünnungsmittel. Die Reaktion wird zwischen -10 und +50 °C, vorzugsweise zwischen 20 und 40 °C durchgeführt. Es wird auf 1 Mol Verbindung der Formel II ca. 1-1,5 Mol, bevorzugt etwa 1,2 Mol Aryl, bevorzugt p-Toluolsulfonylchlorid, eingesetzt.

Die Wirkstoffe eignen sich bei günstiger Warmblütertoxizität zur Bekämpfung von tierischen Schädlingen wie Arthropoden, vorzugsweise Insekten und Spinnentieren, die in der Tierhaltung und Tierzucht bei Haus- und Nutztieren sowie Zoo-, Labor-, Versuchs- und Hobbytieren vorkommen. Sie sind dabei gegen alle oder einzelne Entwicklungsstadien der Schädlinge sowie gegen resistente und normal sensible Arten der Schädlinge wirksam.

Durch die Bekämpfung der tierischen Schädlinge sollen Krankheiten und deren Übertragung, Todesfälle und Leistungsminderungen (z.B. bei der Produktion von Fleisch, Milch, Wolle, Häuten, Eiern) vermindert werden, so daß durch den Einsatz der Wirkstoffe eine wirtschaftlichere und einfachere Tierhaltung möglich ist bzw. in bestimmten Gebieten erst möglich wird.

Zu den Schädlingen gehören:

Aus der Ordnung der Anoplura z.B. Haematopinus spp., Linognathus spp., Solenopotes spp., Pediculus

spp., Pthirus spp.;
aus der Ordnung der Mallophaga z.B. Trimenopon spp., Menopon spp., Eomenacanthus spp., Menacanthus spp., Trichodectes spp., Felicola spp., Damalinea spp., Bovicola spp.;
aus der Ordnung der Diptera z.B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp..
Aus der Ordnung der Siphonaptera z.B. Ctenocephalides spp., Echidnophaga spp., Ceratophyllus spp..
Aus der Ordnung der Metastigmata z.B. Hyalomma spp., Rhipicephalus spp., Boophilus spp., Amblyomma spp., Haemaphysalis spp., Dermacentor spp., Ixodes spp., Argas spp., Ornithodorus spp., Otobius spp.;
aus der Ordnung der Mesastigmata z.B. Dermanyssus spp., Ornithonyssus spp., Pneumonyssus spp..
Aus der Ordnung der Prostigmata z.B. Cheyletiella spp., Psorergates spp., Myobia spp., Demodex spp., Neotrombicula spp.;
aus der Ordnung der Astigmata z.B. Acarus spp., Myocoptes spp., Psoroptes spp., Chorioptes spp., Otodectes spp., Sarcoptes spp., Notoedres spp., Knemidocoptes spp., Neoknemidocoptes spp., Lytodites spp., Laminosioptes spp..

Zu den Haus- und Nutztieren gehören Säugetiere wie z.B. Rinder, Pferde, Schafe, Schweine, Ziegen, Hunde, Katzen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Fasanen, Enten.

Zu Labor- und Versuchstieren gehören z.B. Mäuse, Ratten, Meerschweinchen, Goldhamster, Hunde und Katzen.

Zu den Hobbytieren gehören z.B. Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Boli, Tränken, Granulaten, oral applizierbare Lösungen, Suspensionen oder Emulsionen, medikiertem Futter oder Trinkwasser. Die dermale Anwendung geschieht z.B. in Form des Tauchens (Dippen), Sprühens (Sprayen) oder Aufgießens (pour-on und spot-on) und des Einpuderns. Die parenterale Anwendung geschieht z.B. in Form der Injektion (z.B. intramusculär, subcutan, intravenös) oder durch Implantate.

Besonders hervorgehoben seien die Zubereitungen zur dermalen Anwendung. Dazu gehören Lösungen, Suspensions- und Emulsionskonzentrate sowie Mikroemulsionen, die vor Anwendung mit Wasser verdünnt werden, Aufgußformulierungen, Pulver und Stäube, Aerosole und wirkstoffhaltige Formkörper, sowie dustbags, back rubbers.

Diese Zubereitungen werden in bekannter Weise hergestellt, z.B. durch Vermischen des Wirkstoffs mit Streckmitteln, also z.B. flüssigen Lösungsmitteln, gegebenenfalls unter Verwendung von oberflächenaktiven Mitteln, also Emulgiermitteln und/oder Dispergiermitteln. Im Falle der Benutzung von Wasser als Streckmittel können z.B. auch organische Lösungsmittel als Hilfslösungsmittel verwendet werden.

Zu den flüssigen Verdünnungsmitteln zählen neben Wasser Alkohole wie Methanol, Ethanol, Isopropanol, n-Butanol, Amylalkohol, Octanol;
Glykole wie Propylenglykol, 1,3-Butylenglykol, Ethylglykol, Dipropylenglykolmonomethylether;
Glycerin;
aromatische Alkohole wie Benzylalkohol;
Carbonsäureester wie z.B. Ethylacetat, Benzylbenzoat, Butylacetat, Propylencarbonat, Milchsäureethylester;
aliphatische Kohlenwasserstoffe wie Paraffine, Cyclohexan, Methylenchlorid, Ethylenchlorid;
aromatische Kohlenwasserstoffe wie Xylol, Toluol, Alkylnaphthaline, Chlorbenzole;
Ketone wie z.B. Aceton und Methylethylketon, Methylisobutylketon, Cyclohexanon;
natürliche und synthetische Mono- und Triglyceride mit natürlichen Fettsäuren wie Baumwollsaatöl, Erdnußöl, Maiskeimöl, Olivenöl, Ricinusöl, Sesamöl;
weiterhin Dimethylsulfoxid, Dimethylacetamid, Dimethylformamid, N-Methylpyrrolidon, Dioxan, 2,2-Dimethyl-4-oxymethyl-1,3-dioxolan.

Zu den oberflächenaktiven Stoffen zählen:
Emulgatoren und Netzmittel wie anionaktive Tenside, z.B. Alkylsulfonate, Alkylsulfate, Arylsulfonate, Na-Laurylsulfate, Fettalkoholethersulfate, Mono/Dialkylpolyglykoletherorthophosphorsäureester-Monoethanolaminsalz, Calciumalkylarylsulfonat;
kationaktive Tenside, z.B. Cetyltrimethylammoniumchlorid;

ampholytische Tenside, z.B. Di-Na-N-lauryl-betaiminodipropionat oder Lecithin;
nichtionogene Tenside, z.B. polyoxyethyliertes Ricinusöl, polyoxyethyliertes Sorbitan-Monooleat, polyoxyethyliertes Sorbitan-Monostearat, Glycerinmonostearat, Polyoxyethylenstearat, Alkylphenolpolyglykolether, polyoxyethyliertes Sorbitanmonopalmitat, Polyoxyethylenlaurylether, Polyoxyethylen-oleylether, Polyoxyethylenmannitanmonolaurat, Alkylpolyglykolether, Oleylpolyglykolether, Dodecylpolyglykolether, ethoxyliertes Nonylphenol, Isooctylphenolpolyethoxyethanol.

Die Zubereitungen können außerdem enthalten:
Haftvermittler, z.B. Carboxymethylcellulose, Methylcellulose und andere Cellulose- und Stärke-Derivate, Polyacrylate, Alginate, Gelatine, Gummi arabicum, Polyvinylpyrrolidon, Polyvinylalkohol, Copolymere aus Methylvinylether und Maleinsäureanhydrid, Polyethylenglykole, Paraffine, Öle, Wachse, hydriertes Rizinusöl, Lecithine und synthetische Phospholipide.

Die Zubereitungen können Farbstoffe wie anorganische Pigmente, z.B.Eisenoxid, Titanoxid, Ferrocyanblau und organische Farbstoffe, wie Alizarin-, Azo- und Metallphthalocyaninfarbstoffe enthalten.

Die Zubereitungen können Spreizmittel enthalten, z.B. Silikonöle verschiedener Viskosität, Fettsäureester wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester, Dibutylphthalat, Adipinsäurediisopropylester, letzterem verwandte Estergemische u.a.;
Triglyceride wie Capryl/Caprinsäuretriglycerid, Triglyceridgemische mit Pflanzenfettsäuren der Kettenlänge $C_8$-$C_{12}$ oder anderen speziell ausgewählten natürlichen Fettsäuren, Partialglyceridgemische gesättigter oder ungesättigter evtl. auch hydroxylgruppenhaltige Fettsäuren, Monodiglyceride der $C_8$/$C_{10}$-Fettsäuren und andere; Fettalkohole wie Isotridecylalkohol, 2-Octyldodecanol, Cetylstearyalkohol, Oleylalkohol.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind gegebenenfalls gebrochene und fraktionierte, z.B. synthetische und natürliche Gesteinsmehle wie Kaoline, Talkum, Kreide, Quarz, Diatomeenerde, Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken, Sägemehl.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in Form ihrer oben erwähnten festen oder flüssigen Formulierungen auch eingekapselt vorliegen.

Die Wirkstoffe können auch in Form eines Aerosols angewendet werden. Dazu wird der Wirkstoff in geeigneter Formulierung unter Druck fein verteilt.

Es kann auch vorteilhaft sein, die Wirkstoffe in Formulierungen anzuwenden, die den Wirkstoff verzögert frei geben. Als solche seien wirkstoffhaltige Formkörper wie z.B. Platten, Bänder, Streifen, Halsbänder, Ohrmarken, Schwanzmarken, Gliedmaßenbänder, Halfter, Markierungsvorrichtungen genannt. Als solche seien auch wirkstoffhaltige Implantate und Boli genannt.

Die Verabreichung der Wirkstoffe kann auch zusammen mit dem Futter und/oder dem Trinkwasser erfolgen.

Die Wirkstoffe können in den Formulierungen allein oder in Mischung mit anderen Wirkstoffen oder Synergisten vorliegen.

Direkt angewendete Formulierungen enthalten zwischen $10^{-7}$ und 5 Gewichtsprozent, bevorzugt zwischen $10^{-4}$ und 1 Gewichtsprozent Wirkstoff.

Formulierungen die erst nach weiterer Verdünnung angewendet werden enthalten 1 - 95 Gewichtsprozent, bevorzugt 5 - 90 Gewichtsprozent Wirkstoff.

<u>Beispiel A</u>

Test mit Boophilus microplus resistent

Lösungsmittel:

35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: Beispiel 1.

## Beispiel B

Test mit Psoroptes ovis

Emulgator:

35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die gewünschte Konzentration.

Etwa 10 - 25 Psoroptes ovis werden in 1 ml der zu testenden Wirkstoffzubereitung gebracht, die in Tablettennester einer Tiefziehverpackung pipettiert wurden. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: Beispiel 1.

## Herstellungsbeispiele

Beispiel 1

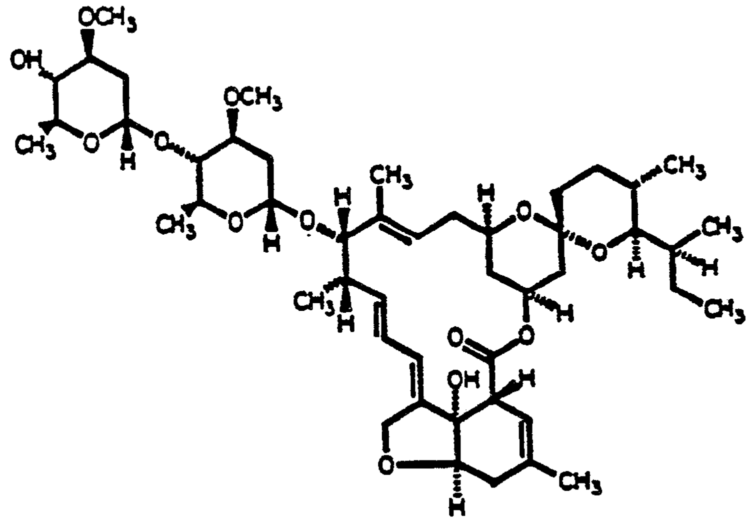

Ia

1,2 g der in 5-Stellung bromsubstituierten Verbindung der Formel IIa werden in 10 ml abs. Toluol gelöst, mit 2,0 ml Tributylzinnhydrid versetzt und mit 200 mg Azobisisobutyronitril versetzt. Man erwärmt auf 50 ° C. Nach 2 h wird das Gemisch auf eine mit Toluol equilibrierte Säule mit Kieselgel 60 (Merck 230-400 mesh) gegeben und mit Toluol die zinnorganischen Verbindungen eluiert. Die Verbindung der Formel Ia wird dann mit Toluol/Ethylacetat 8:1 eluiert.

Beispiel 2

Ausgehend von 1,2 g der in 5-Stellung chlorsubstituierten Verbindung gemäß Formel II a wird analog zu Beispiel 1 die Verbindung der Formel I a erhalten.

Herstellung der Ausgangsverbindungen gemäß Verfahren 6 und 4:

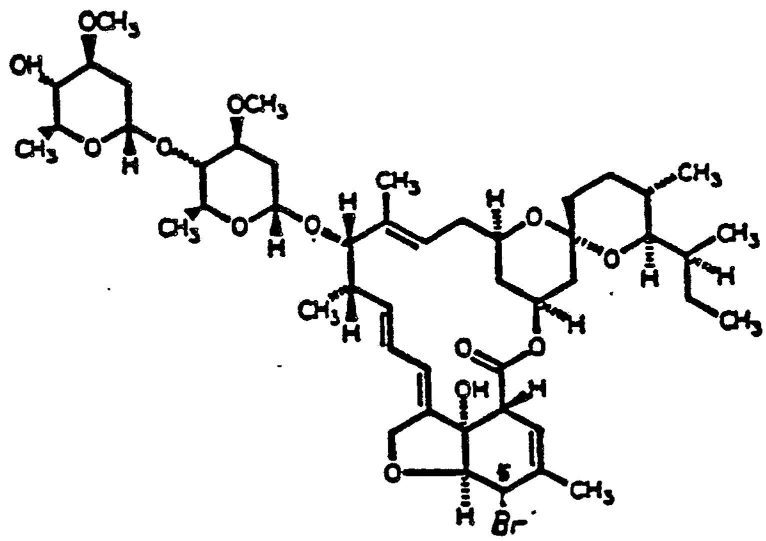

IIa

Beispiel a

7,0 g in 5-Stellung OH substituierten Verbindung der Formel IIa werden in 50 ml trockenem ethanolfreiem Chloroform gelöst, mit 6,5 ml abs. Pyridin versetzt und auf -25°C gekühlt. Zu dieser Lösung wird langsam eine Lösung aus 1,0 ml Methansulfonylchlorid in 5,0 ml abs. Chloroform zugetropft. Nach ca. 2 h ist die Umsetzung beendet. Überschüssiges Methansulfonylchlorid wird durch Zugabe von 1,0 ml Methanol zerstört. Der Ansatz wird mit

Der erhaltene Rohsirup wird in 50 ml trockenem Chloroform gelöst und mit 10 g Tetrabutylammoniumbromid versetzt. Nach ca. 4 h ist die Umsetzung beendet (DC-Nachweis: Zuerst mit einer 0,1 prozentigen ethanolischen Fluoresceinlösung ansprühen, trocknen und mit Essigsäure/Perhydrol 1:1 ansprühen, dann Wärmebehandlung). Zur Aufarbeitung wird der Ansatz eingeengt, in Toluol aufgenommen und erneut eingeengt. Der Rückstand wird in Toluol/Aceton 8:1 chromatographiert. Man erhält 5 g der in 5-Stellung bromsubstituierten Verbindung der Formel IIa.

Beispiel b

3,0 g der in 5 Stellung OH-substituierten Verbindung der Formel IIa werden in 25 ml abs. Chloroform gelöst und unter Eiskühlung mit 3,0 g Tosylchlorid und 3,0 g Dimethylaminopyridin versetzt. Sobald die Umsetzung zum 5-O-Monotosylat beendet ist wird überschüssiges Tosylchlorid mit Methanol zerstört. Anschließend wird der Ansatz noch 5 h bei Raumtemperatur belassen. Zur Aufarbeitung wird der Ansatz zuerst mit wässrigem Phosphatpuffer und dann mit Wasser gewaschen. Die organische Phase wird über Magnesiumsulfat getrocknet und eingeengt. Zur Reinigung wird an Kieselgel in Toluol/Aceton 5:1 chromatographiert. Es wird die in 5-Stellung chlorsubstituierte Verbindung der Formel II a erhalten.

## Ansprüche

1. Neue Avermectin-Derivate der Formel I

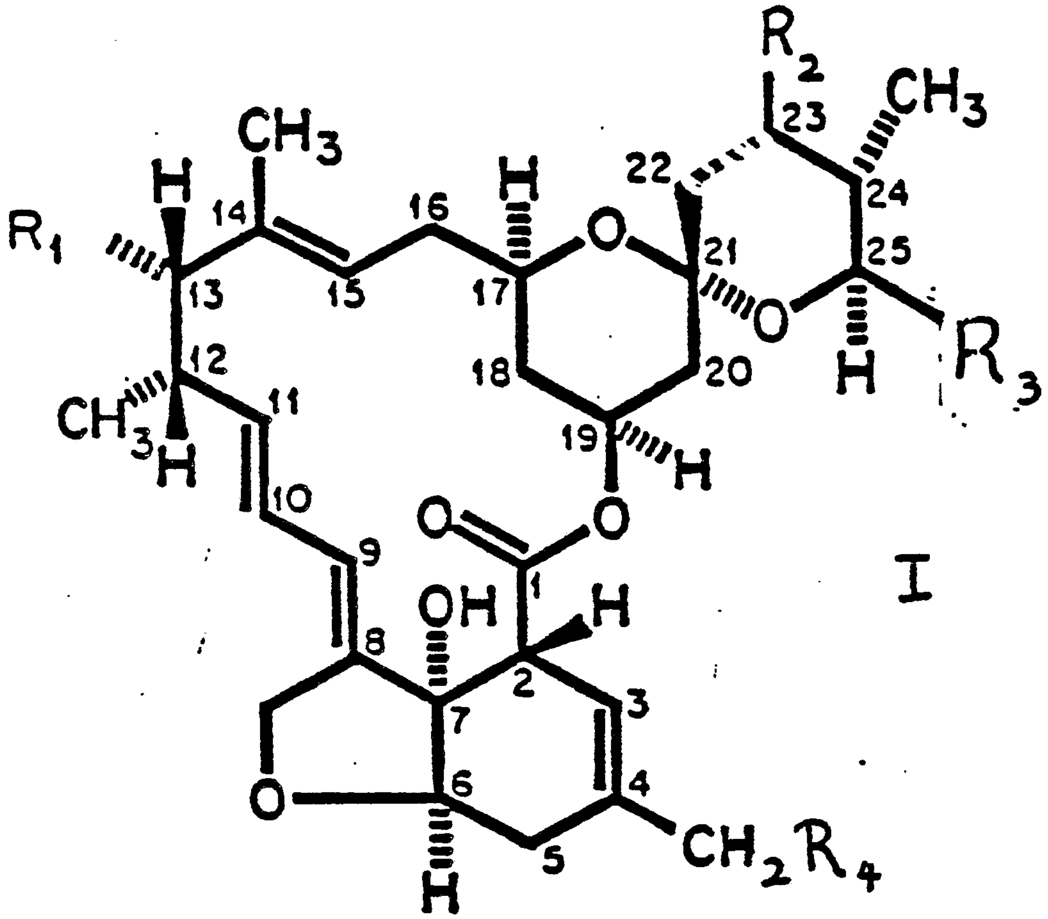

I

in welcher
$R^1$ für Wasserstoff, OH, $C_{1-5}$-Alkanoyloxy, α-L-Oleandrosyloxy, α-L-Oleandrosyl-α-L-oleandrosyloxy, 4′-$C_{1-5}$-Alkanoyl-α-L-Oleandrosyloxy, 4″-$C_{1-5}$-Alkanoyl-α-L-Oleandrosyl-α-L-oleandrosyloxy steht,
$R^2$ für Wasserstoff, OH, $C_{1-5}$-Alkanoyloxy steht, $R^2$ steht für Wasserstoff, wenn zwischen C22 und C23 eine Doppelbindung ist,
$R^3$ für geradkettiges oder verzweigtes Alkyl oder Alkenyl steht,
$R^4$ für Wasserstoff, OH, $C_{1-5}$-Alkanoyloxy, Heterocyclylcarbonyloxy steht, die Bindung zwischen den C-Atomen C22 und C23 eine Einfach- oder eine Doppelbindung ist und die Doppelbindung des Cyclohexenringes zwischen den C-Atomen C3 und C4 oder zwischen den C-Atomen C4 und C5 stehen kann.

2. Verfahren zur Herstellung der Verbindungen der Formel I

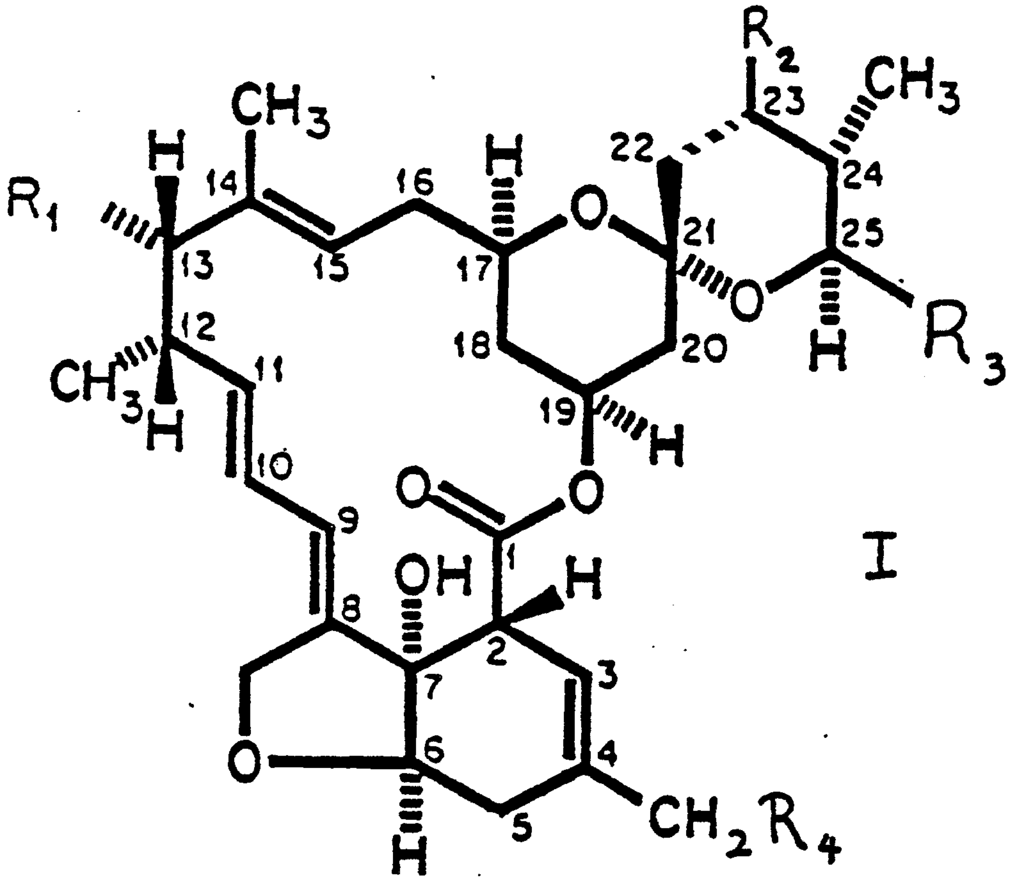

I

in welcher

$R^1$ für Wasserstoff, OH, $C_{1-5}$-Alkanoyloxy, α-L-Oleandrosyloxy, α-L-Oleandrosyl-α-L-oleandrosyloxy, 4′-$C_{1-5}$-Alkanoyl-α-L-Oleandrosyloxy, 4″-$C_{1-5}$-Alkanoyl-α-L-Oleandrosyl-α-L-oleandrosyloxy steht,

$R^2$ für Wasserstoff, OH, $C_{1-5}$-Alkanoyloxy steht, $R^2$ steht für Wasserstoff, wenn zwischen C22 und C23 eine Doppelbindung ist,

das dadurch gekennzeichnet ist, daß man Verbindungen der Formel II

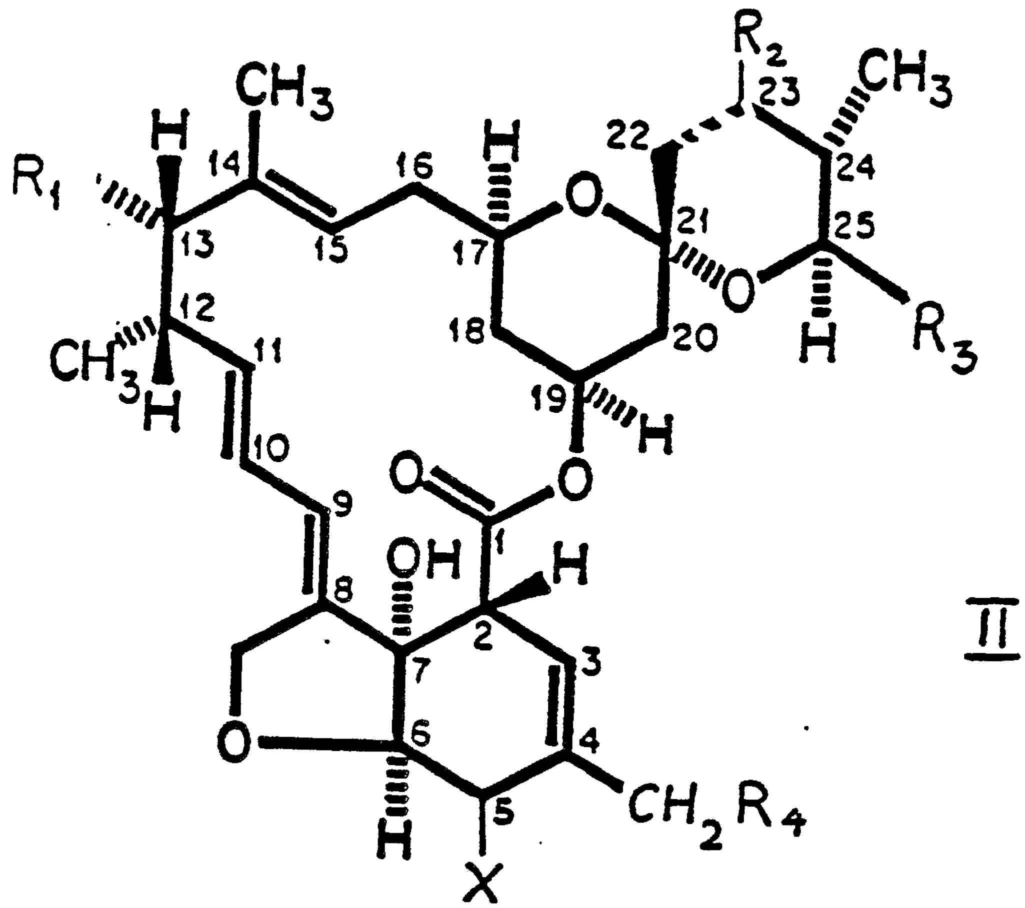

II

in welcher
$R^1$, $R^2$, $R^3$, $R^4$ die oben angegebene Bedeutung haben und
X für Halogen steht
reduziert.

3. Neue Verbindungen der Formel II

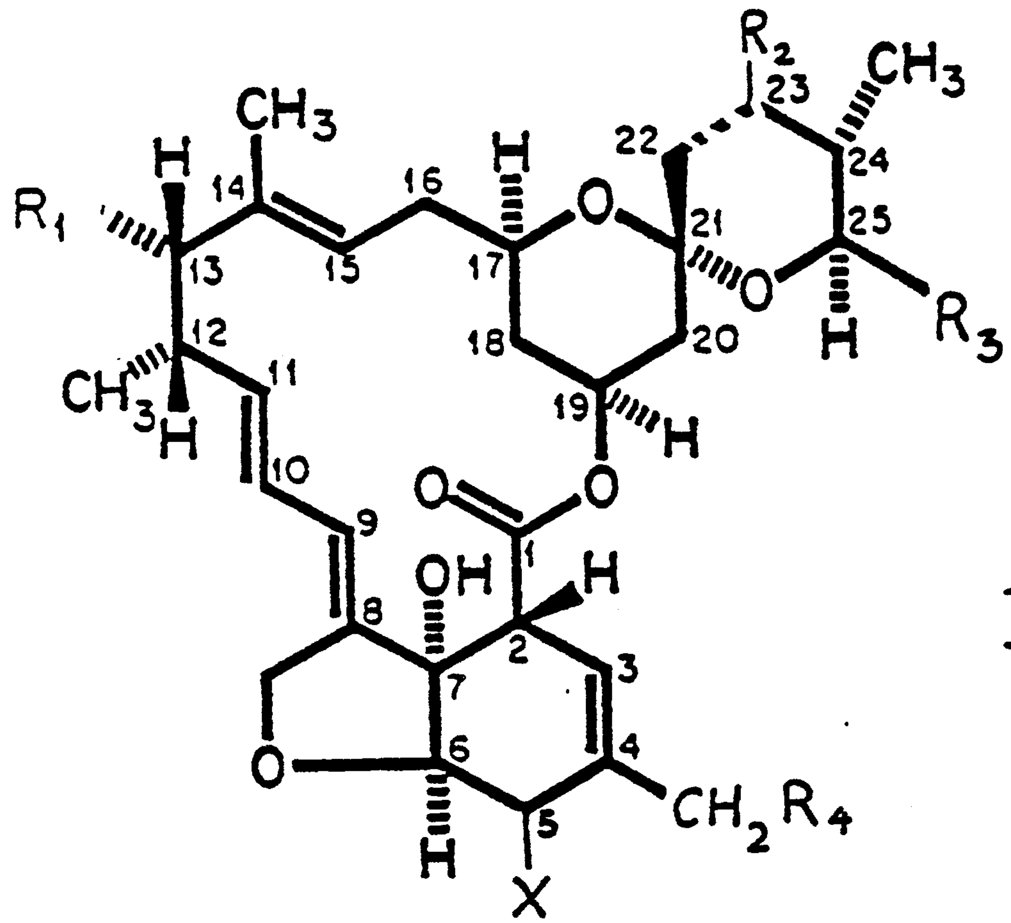

II

in welcher
$R^1$ für Wasserstoff, OH, $C_{1-5}$-Alkanoyloxy, α-L-Oleandrosyloxy, α-L-Oleandrosyl-α-L-oleandrosyloxy, 4′-$C_{1-5}$-Alkanoyl-α-L-Oleandrosyloxy, 4″-$C_{1-5}$-Alkanoyl-α-L-Oleandrosyl-α-L-oleandrosyloxy steht,
$R^2$ für Wasserstoff, OH, $C_{1-5}$-Alkanoyloxy steht, $R^2$ steht für Wasserstoff, wenn zwischen C22 und C23 eine Doppelbindung ist,
$R^3$ für geradkettiges oder verzweigtes Alkyl oder Alkenyl steht,
$R^4$ für Wasserstoff, OH, $C_{1-5}$-Alkanoyloxy, Heterocyclylcarbonyloxy steht, die Bindung zwischen den C-Atomen C22 und C23 eine Einfach- oder eine Doppelbindung ist und die Doppelbindung des Cyclohexenringes zwischen den C-Atomen C3 und C4 oder zwischen den C-Atomen C4 und C5 stehen kann,
X für Halogen steht.

4. Verfahren zur Herstellung der Verbindungen der Formel II gemäß Aspruch 3 (oben) dadurch gekennzeichnet, daß man Verbindungen der Formel II in welcher
X für den Rest $-OSO_2-R^5$ steht wobei
$R^5$ für Alkyl, Halogenalkyl, Aryl, substituiertes Aryl steht,
mit Halogeniden umsetzt, oder Verbindungen der Formel II, in welcher X für OH steht, mit Arylsulfonylhalogeniden in Gegenwart von Dimethylaminopyridin umsetzt.

5. Schädlingsbekämpfungsmittel, gekennzeichnet durch einen Gehalt an mindestens einem Avermectin-Derivat der Formel (I), gemäß Anspruch 1.

6. Verwendung von Avermectin-Derivaten der Formel (I) gemäß Anspruch 1 zur Bekämpfung von Schädlingen.

7. Verwendung von Avermectin-Derivaten der Formel (I) gemäß Anspruch 1 zur Herstellung von Schädlingsbekämpfungsmitteln.